# EUROPÄISCHE PATENTANMELDUNG

(11) **EP 2 243 768 A1**
(43) Veröffentlichungstag der Anmeldung: **27.10.2010**
(21) Anmeldenummer: 09158489.6
(22) Anmeldetag: 22.04.2009
(51) Int. Cl.: C07C 245/20, C07C 37/14, C07C 39/15, C07C 39/19

(54) **Phenolisches Diazoniumsalz, Verfahren zu dessen Herstellung und dessen Verwendung**

(71) Anmelder: Zylum Beteiligungsgesellschaft mbH & Co. Patente II KG, 12529 Schönefeld / Waltersdorf (DE)
(72) Erfinder: Böge, Nicolas, Dr., 20149 Hamburg (DE); Kreipl, Andreas, Dr., 20359 Hamburg (DE); Schmidt, Bernd, Prof. Dr., 14482 Potsdam (DE); Hölter, Frank, 14473 Potsdam (DE); Berger, René, 14471 Potsdam (DE)
(74) Vertreter: Zech, Stefan Markus

(57) **Zusammenfassung**

Beschrieben wird ein phenolisches Diazoniumsalz der allgemeinen Formel (I)

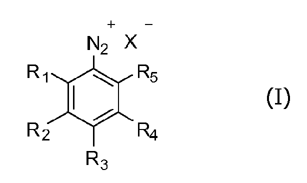

Dieses phenolische Diazoniumsalz ist **dadurch gekennzeichnet, dass** mindestens einer von R¹, R², R³, R⁴ oder R⁵ eine Hydroxy- gruppe ist und die anderen Substituenten, unabhängig voneinander, Wasserstoff, Halogen, eine Alkyl-, Alkenyl-, Aryl-, Alkoxy-, Aryloxy-, Nitro-, Cyano-, Hydroxy-, Acetyl- und/oder Diazogruppen darstellen, und X BF₄, Cl, F, SO₃CH₃, CO₂CH₃, PF₆, ClO₂CH₃ oder ClO₄ darstellen. Ferner betrifft die Erfindung ein Verfahren zur Herstellung dieses Diazoniumsalzes, wonach als Ausgangsverbindung ein benzolisches Amid, das bis auf die Diazofunktion die gleichen Substituenten R¹, R², R³, R⁴ oder R⁵ aufweist, eingesetzt wird und dieses zunächst hydrolytisch gespalten und das so erhaltene Amin mit einem anorganischen Nitritsalz diazotiert und gegebenenfalls isoliert wird. Der besondere Wert des beschriebenen phenolischen Diazoniumsalzes erweist sich in seiner Verwendung in Kreuzkupplungsreaktionen. Die Reaktionsprodukte bei den Kreuzkupplungsreaktionen lassen sich in einfacher und wirtschaftlicher Weise, insbesondere in hoher Ausbeute herstellen. Darüber hinaus hat es sich gezeigt, dass mit dem Stand der Technik verbundenen Probleme der Regioselektivität weitgehend minimiert werden.

## Beschreibung

Die Erfindung betrifft ein phenolisches Diazoniumsalz, das eine ungeschützte phenolische Gruppe aufweist. Die Herstellung solcher Salze beruht insbesondere auf dem Grundgedanken, dass ein Amid hydrolytisch gespalten und das erhaltene Amin diazotiert wird. Das phenolische Diazoniumsalz eignet sich insbesondere als Substrat in einer Kreuzkupplungsreaktion.

Phenolische Diazoniumsalze sind im Stand der Technik bekannt. So wird in der DE 10 2006 053 064 A1 zum Beispiel offenbart p-Benzyloxyphenyldiazoniumtetrafluoroborat. Diese Verbindung weist jedoch für die Hydroxygruppe des Grundgerüsts eine Schutzgruppe auf. Die Schutzgruppen sollen derart ausgewählt werden, dass sie einerseits der hydrolytischen Spaltung der Amid-Gruppe und andererseits der Diazotierungsreaktion widerstehen. Derartige Schutzgruppen sind dem Fachmann an sich bekannt und werden beispielsweise beschrieben in "Protective Groups in Organic Chemistry" von Theodora W. Greene, Wiley Verlag, 1981.

Die bezeichnete Fluorborat-Verbindung stellt eine reaktive Ausgangsverbindung dar, die in an sich bekannter Weise umgesetzt werden kann. Die DE 10 2006 053 064 A1 weist hier beispielhaft auf die Japp-Klingemann-Reaktion, Desaminierung, Sandmeyer-Reaktion, Schiemann-Reaktion, Meerwein-Reaktion und Gomberg-Bachmann-Reaktion hin, wobei die in diesem Stand der Technik bezeichneten Diazoniumsalze insbesondere für die Heck-Reaktion geeignet sein sollen. Die oben beschriebene bekannte Lehre ist aus folgenden Gründen nachteilig: Vor der Überführung in ein Diazoniumsalz muß die phenolische Ausgangsverbindung mit einer Schutzgruppe versehen werden. Das Salz wird dann in situ in Kreuzkupplungsreaktionen eingesetzt, bevor daran anschließend die Abspaltung der Schutzgruppe erfolgt. Ersichtlich ist dieses Verfahren aufwendig. Ein ähnlicher technischer Sachverhalt ergibt sich aus der EP 1 253 466 B1.

Alternative Herstellungsverfahren für Phenol-enthaltende Zielverbindungen, bei denen die Verwendung von Schutzgruppen vermieden werden kann, sind ebenfalls aus dem Stand der Technik bekannt.

So beschreibt die Literaturstelle "Tetrahedron Lett.", 1979, 657-660, ein Verfahren zur Synthese von Biarylen, die mechanistisch wie eine Gomberg-Bachmann-Reaktion bzw. in der intramolekularen Variante wie eine Pschorr-Cyclisierung abläuft. Ausgegangen wird dabei von einem Phenyldiazoniumtetrafluoroborat, das in Gegenwart stöchiometrischer Mengen einer Titan-III-Verbindung unter Abspaltung von Stickstoff reduziert wird, und das entstehende Arylradikal ein zur Reaktion bereitgestelltes Phenol bevorzugt in Ortho-Position angreift. Die Regioselektivität der beschriebenen Reaktion ist jedoch nicht perfekt. Anschließend erfolgt die Oxidation des neuen Radikals unter Abspaltung eines Protons und Bildung des Biaryls. Wesentliche Nachteile dieser Methode sind zum einen die Verwendung eines Äquivalents einer luftempfindlichen und teuren Titan-III-Verbindung, was in der Folge zur Entstehung von äquimolaren Mengen an Metallabfällen (Titan-IV-Verbindungen) führt, die aufwendig abgetrennt werden müssen. Zum anderen erfolgt die Reaktion in Gegenwart einer Säure, so dass die Toleranz gegenüber funktionellen Gruppen eingeschränkt ist. Schließlich beträgt die Ausbeute maximal lediglich 31%.

Ein weiterer relevanter Stand der Technik ergibt sich aus der US 2003/0120124 A1. Diese beschreibt die Verwendung von Diazoniumsalzen als Substrat in Kupplungsreaktion. Hier wird beispielsweise eine Kupplungsreaktion vorgeschlagen, bei der Phenyltrimethylsilan mit Aryldiazoniumsalzen umgesetzt wird, was dem Typ einer Hiyama-Kupplung entspricht. Dieser Stand der Technik stellt sich nicht dem Problem, bei der Kupplungsreaktion phenolische Verbindungen einzusetzen und deren Hydroxygruppe, wie im Stand der Technik vorgeschlagen, mit einer Schutzgruppe zu versehen, um danach erst die gewünschten Kupplungsreaktionen ablaufen zu lassen. Analog wurde in "Advanced Synthesis & Catalysis", 2008, 350 (10), 1577-86, die Verwendung von 2-Nitro-substituierten Phenyldiazoniumsalzen als Substrat in Suzuki-Kreuzkupplungen beschrieben.

Ausgehend von dem vorstehend geschilderten Stand der Technik liegt der Erfindung die Aufgabe zugrunde, einen technischen Vorschlag zu unterbreiten, wie Kreuzkupplungen einfacher und mit verbesserter Ausbeute angewandt werden können, ohne dass die Hydroxylgruppe bei hydroxylgruppenhaltigen aromatischen Edukten mit einer Schutzgruppe versehen werden muß. Auch soll die Erfindung gegenüber demjenigen Stand Vorteile bieten, bei denen die Hydroxylgruppen nicht im aromatischen Diazoniumsalz, sondern im Kupplungspartner enthalten sind, die jedoch im Rahmen der Kupplungsreaktion eine schlechte Regioselektivität zeigen.

Erfindungsgemäß wird diese Aufgabe gelöst durch ein phenolisches Diazoniumsalz der allgemeinen Formel (I) 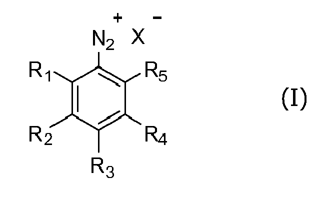 das dadurch gekennzeichnet ist, dass mindestens einer von R¹, R², R³, R⁴ oder R⁵ eine Hydroxygruppe ist und die anderen Substituenten, unabhängig voneinander, Wasserstoff, Halogen, eine Alkyl-, Alkenyl-, Aryl-, Alkoxy-, Aryloxy-, Nitro-, Cyano-, Hydroxy-, Acetyl- und/oder Diazogruppen darstellen, und X BF₄, Cl, F, SO₃CH₃, CO₂CH₃, PF₆, ClO₂CH₃ oder ClO₄ darstellen.

Die obige Darstellung umfasst verschiedene konkrete Gruppen. Diese sind nicht weitergehend auszugestalten. Andererseits umfasst sie jedoch auch allgemeine Begriffe, die im Rahmen der Erfindung vorteilhaft konkretisiert werden können. So hat es sich als zweckmäßig erwiesen, dass die Alkylgruppe eine geradkettige oder verzweigte (C₁-C₆)-Alkylgruppe, eine (C₃-C₇)-Cycloalkylgruppe, die Alkenylgruppe eine geradkettige oder verzweigte (C₁-C₆)-Alkenylgruppe, die Alkoxygruppe eine geradkettige oder verzweigte (C₁-C₆)-Alkyloxygruppe, die Arylgruppe eine Benzyl-, 4-Methoxybenzyl-, 4-Nitrobenzyl-, Phenyl- oder 4-Methoxyphenyl-Gruppe darstellt und Halogen Fluor, Chlor oder Brom darstellen.

Besonders bevorzugt ist es, wenn R³ eine Hydroxygruppe ist. In Einzelfällen ist es auch vorteilhaft, wenn R¹, R², R⁴ und R⁵ jeweils Wasserstoffe sind. Allerdings ist es hierzu komplementär in Einzelfällen auch vorteilhaft, wenn neben der Hydroxy- und der Diazogruppe mindestens ein weiterer Substituent in der Verbindung gemäß Formel (I) vorliegt, der kein Wasserstoff ist.

Ein weiteres wesentliches Merkmal der Verbindung gemäß Formel (I) ist das Anion X, bei dem es sich vorzugsweise um Tetrafluoroborat handelt.

Die nachfolgenden Beispiele zeigen, dass die Verbindungen, die von der vorstehenden Formel (I) erfasst werden, die gestellte Aufgabe in überraschend günstigem Ausmaße lösen. Dies gilt sowohl für die Ausbeute als auch für die einfache Verfahrensführung sowie für den oben angesprochenen Gesichtspunkt der Regioselektivität.

Das erfindungsgemäße Verfahren ist **dadurch gekennzeichnet, dass** als Ausgangsverbindung ein benzolisches Amid, das bis auf die Diazofunktion die gleichen Substituenten R¹, R², R³, R⁴ oder R⁵ aufweist, eingesetzt wird und dieses zunächst hydrolytisch gespalten und das so erhaltene Amin anschließend mit einem anorganischen Nitritsalz diazotiert und gegebenenfalls isoliert wird.

Um besonders vorteilhafte Verbindungen der Formel (I) zu erhalten, wird das diazotierte Zwischenprodukt durch Zugabe eines Komplexanionsalzes mit einem Anion in Form von BF₄⁻, PF₆⁻ und/oder ClO4⁻ in das Diazoniumsalz überführt, wenn bei der Diazotierung ein Anion eingesetzt wird, das diesen Komplex Anionen nicht entspricht. Als Beispiel sei die Diazotierung mit einer Halogenwasserstoffsäure angegeben, wie beispielsweise Chlorwasserstoffsäure.

Die angegebene hydrolytische Spaltung ist dann besonders zweckmäßig ausgestaltet, wenn sie mit einer Mineralsäure oder Fluoroborsäure durchgeführt wird. Dabei ist besonders vorteilhaft, wenn die hydrolytische Spaltung in einem alkoholischen Lösungsmittel, das eine Mineralsäure, insbesondere Chlorwasserstoffsäure, oder Fluoroborsäure enthält, durchgeführt wird, wobei der gewählte Alkohol zweckmäßigerweise ein C¹-C₄-Alkohol darstellt. Bevorzugt ist hierbei das alkoholische Lösungsmittel Methanol, Ethanol, n-Propanol und/oder Isopropanol.

Bei der Wahl der Temperatur der hydrolytischen Spaltung ist der Fachmann nicht relevant eingeschränkt. Es ist allerdings bevorzugt, wenn die hydrolytische Spaltung des angesprochenen Amids bei einer Temperatur zwischen etwa 20 und 110°C, insbesondere zwischen etwa 50 und 80°C, durchgeführt wird. Gleichermaßen nicht wesentlich beschränkt ist die Temperatur, die bei der Diazotierung gewählt werden sollte. Sie liegt zweckmäßigerweise zwischen etwa -10 und +10°C, insbesondere zwischen etwa -5 und +5°C. Besonders vorteilhaft ist es, wenn die Diazotierung in der Reaktionsmischung vorgenommen wird, in der bereits die hydrolytische Spaltung vollzogen wurde, d.h. keine zwischengeschaltete Isolierung des Amins erfolgt.

Der besondere Vorteil des erfindungsgemäßen phenolischen Diazoniumsalzes, wie es vorstehend dargestellt wurde, liegt in der Verwendung als Substrat in mehr oder weniger beliebigen Kreuzkupplungsreaktionen, wobei die bereits vorstehenden angesprochenen Vorteile in überraschend günstigem Ausmaß erzielt werden. Lediglich beispielhaft und nicht beschränkend seien für die Kreuzkupplungsreaktionen angeführt die Heck-, Suzuki-, Stille-, Negishi-, Hiyama- oder Kumada-Kupplung. In diesem Zusammenhang sei angemerkt, dass die Heck-Kupplung eigentlich nicht unter den Begriff "Kreuzkupplung" fällt. Sie soll aber trotzdem im Zusammenhang mit der hier beschriebenen Verwendung als eine solche verstanden werden.

Dem Fachmann ist es zwar problemlos möglich, anhand seines Fachwissens unter Nutzung der erfindungsgemäßen phenolischen Diazoniumsalze diese Kreuzkupplungen mit Erfolg durchzuführen. Es hat sich jedoch gezeigt, dass die Kreuzkupplungsreaktion besonders vorteilhaft nach einem Verfahren durchgeführt wird, das umfasst:
(a) das Mischen eines in den Ansprüchen 1 bis 6 definierten phenolischen Diazoniumsalzes mit einem Kupplungspartner in Anwesenheit eines Katalysators, wobei der Kupplungspartner eine Verbindung der allgemeinen Formel (II) 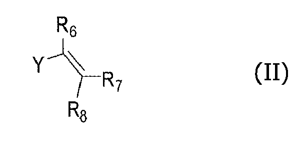 darstellt, R⁶, R⁷ und R⁸, die gleich oder verschieden sind, Wasserstoff, Carboxyalkylreste, Carboxyarylreste, Alkylreste, Arylreste, Alkoxyreste, Aryloxyreste, wobei die Reste jeweils Si, N, S, O, und oder Halogen-Atome enthalten können, darstellen oder R⁶ und R⁷ mit der Doppelbindung einen aromatischen Ring bilden, der mit R⁸ und ein bis vier weiteren Dubstituenten, unabhängig voneinander, ausgewählt aus der Gruppe bestehend aus einer geradkettigen oder verzweigten (C₁-C₆)-Alkylgruppe, einer (C₃-C₇)-Cycloalkylgruppe, einer geradkettigen oder verzweigten (C₁-C₆)-Alkenylgruppe, einer geradkettigen oder verzweigten (C₁-C₆)-Alkoxygruppe, Halogen, der Hydroxygruppe, einer Amino-, Di(C₁-C₆)-alkylamino-, Nitro-, Acetyl-, Cyan-, Benzyl-, 4-Methoxybenzyl, 4-Nitrobenzyl-, Phenyl-, und 4-Methoxyphenylgruppe versehen sein kann und Y = H, -B(OR)₂, -SnR₃, -ZnR, -SiR₃ oder Mg (Halogen) ist, (b) Umsetzen des phenolischen Diazoniumsalzes mit dem Kupplungspartner und der Bildung des kreuzgekuppelten Produkts.
   Wenn hier der Begriff "Katalysator" verwendet wird, heißt das selbstverständlich, dass er in geringen Mengen eingesetzt wird, d.h. in der Regel bis zu 5 Mol-%, bezogen auf das eingesetzte Diazoniumsalz. Mit besonderem Vorteil wird hier ein ÜbergangsmetallKatalysator eingesetzt, bei dem es sich insbesondere um einen Palladium-Katalysator handelt. In besonderem Vorteil wird als Katalysator Pd(OAc)₂ oder Pd₂(dba)₃CHCl₃ eingesetzt.
   Die oben beschriebene erfindungsgemäße Verwendung bzw. das in diese einbezogene Verfahren gestatten vorteilhafte Ausgestaltungen: So ist es zweckmäßig und vorteilhaft, vor der Durchführung des bezeichneten Schritts (b), der das Umsetzen des phenolischen Diazoniumsalzes mit einem Kupplungspartner und die Bildung des kreuzgekuppelten Produktes betrifft, dem Reaktionsgemisch eine Base, insbesondere eine sich in dem Reaktionsgemisch gut lösende Base zur Abpufferung der im Schritt (b) entstehenden Säure zuzugeben. Das Reaktionsgemisch lässt sich dadurch vorteilhaft ausgestalten, dass es ein Lösungsmittel enthält, das auf Methanol, Ethanol, Acetonitril und/oder Wasser beruht. Die Umsetzung bei der Kreuzkupplung ist auch bezüglich der gewählten Temperatur nicht wesentlich eingeschränkt. Vorteilhaft ist es jedoch, wenn die Umsetzung bei einer Temperatur von etwa -10 bis 60°C, insbesondere von etwa 20 bis 30°C durchgeführt wird.
   Die oben im einzelnen dargestellte Erfindung zeigt vielfältige Vorteile gegenüber dem eingangs geschilderten Stand der Technik: Bei der Erdindung können protische Gruppen direkt im Diazoniumsalz eingebracht werden und nicht nur in den Kupplungspartner. Die notwendige Mengen an Metallverbindungen kann auf eine katalytische Menge, d.h. in der Regel 5 Mol-% oder weniger, reduziert werden, was einen deutlichen ökonomischen und ökologischen Vorteil bietet. Die erfindungsgemäß bei den beschriebenen Kreuzkupplungen erzielten Ausbeuten und Selektivitäten sind wesentlich besser, da die herangezogenen Pdkatalysierten Kreuzkupplungen nach anderen Mechanismen ablaufen bzw. keine freien Radikalen nutzen.

Die Erfindung ist auch deswegen attraktiv, da freie Phenole sehr häufig als Strukturelement in Wirk- und Naturstoffen anzutreffen sind, so dass der Schutzgruppenaufwand bei der Synthese erfindungsgemäß merklich reduziert werden kann. So kommen in der Natur zahlreiche Derivate E-konfigurierter Hydroxystilbene vor, wie z. B. Resveratrol, Pinosylvin und Astringenin. Diesen Stoffen ist es gemeinsam, dass sie eine stark desinfizierende Wirkung haben. Erfindungsgemäß lassen sich diese nützlichen Naturstoffe einfach und mit hoher Ausbeute herstellen.

Nachfolgend wird die Erfindung anhand von Beispielen noch näher erläutert, ohne darin eine Beschränkung zu sehen.

### Beispiel 1 (Herstellung von 4-Hydroxyphenyldiazoniumtetrafluoroborat)

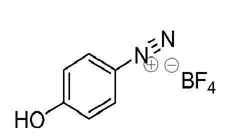

Eine Suspension aus 4-Acetamidophenol (5,0g, 33 mmol) in 3,6 N HBF₄ (15 ml) und Isopropanol (5 ml) wird drei Stunden lang bei 90°C erhitzt bis eine klare Lösung entsteht. Die entstandene Lösung wird auf 0°C gekühlt und NaNO₂ (0,31 g, 4,4 mmol) wird langsam portionsweise hinzugegeben. Die entstehende Suspension wird 30 min lang bei 0°C gerührt. Der Feststoff wird abfiltriert und der Filtrationsrückstand mit kaltem Diethylether (100 ml) gewaschen, was 4-Hydroxyphenyldiazoniumtetrafluoroborat (4,93 g, 24 mmol) in 72 %iger Ausbeute ergibt.
¹H-NMR (300 MHz, DMSO-d₆): δ = 8,31 (d, 2H, J = 9,5 Hz, Ar), 6,73 (d, 2H, J = 9,5 Hz, Ar).
¹³C-NMR (75 MHz, DMSO-d₆, APT): δ = 174,7, 134,4, 121,5, 88,6. IR (cm⁻¹): 3098 (w), 2189 (s, N₂), 1590 (s).
MS (ESI) m/z = 99 (100%), 121 (M⁺, 73%).

### Beispiel 2 (Herstellung von 4-Hydroxy-3-nitrophenyldiazoniumtetrafluoroborat)

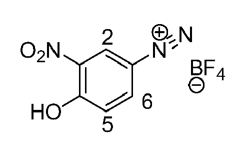

4-Hydroxy-3-nitroacetanilid (2,78 mmol, 500 mg) wird in einem 50 ml Einhalskolben vorgelegt und mit Salzsäure (5 ml, 18%ig) und Ethanol (1 ml) versetzt. Anschließend wird die Suspension sechs Stunden unter Rückfluss erhitzt, wobei sich der Feststoff löst. Die Lösung wird auf 0°C abgekühlt, dabei fällt das Hydrochlorid aus. Bei Zugabe von NaNO₂ (2,78 mmol, 192 mg) über eine Zeitspanne von 15 Minuten löst sich das Hydrochlorid auf, nach zehnminütigem Rühren bei 0°C wird NH₄BF₄ (2,78 mmol, 291 mg) zugegeben. Nach spätestens 15 Minuten fällt ein Feststoff aus, der abgesaugt und mit kaltem Wasser, Ethanol und MTBE (jeweils 20 ml) gewaschen wird. 4-Hydroxy-3-nitrophenyldiazoniumtetrafluoroborat wird als gelber Feststoff mit einer Ausbeute von 57% (1,59 mmol, 400 mg) erhalten.
¹H NMR (300 MHz, DMSO) δ = 8,90 (d, J = 2,9, 1H, 2-H), 7,83 (dd, J = 2,9, 9,8, 1H, 6-H), 6,48 (d, J = 9,8, 1H, 5-H). ¹³C NMR (75 MHz, DMSO) δ = 170,3 (C-4), 140,5 (C-3), 133,6 (Ar), 131,3 (Ar), 128,3 (C-1), 79,5 (Ar). IR (cm⁻¹): 3081 (m), 2166 (s, N₂), 1597 (s), 1326 (s), 1124 (s).
MS (EI) m/z = 63 (100%), 91 (35%), 139 (M⁺, 47%).

### Beispiel 3 (Herstellung von 3-Bromo-4-hydroxyphenyldiazoniumtetrafluoroborat)

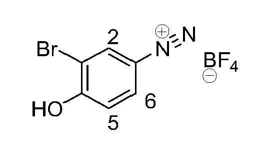

3-Bromo-4-hydroxyacetanilid (2,18 mmol, 500 mg) wird in einem 50 ml Einhalskolben vorgelegt und mit Salzsäure (4 ml, 18%ig) und Ethanol (0,5 ml) versetzt. Anschließend wird die Suspension sechs Stunden unter Rückfluß erhitzt, wobei der Feststoff sich erst löst und wenig später das Hydrochlorid ausfällt. Die Lösung wird auf -10°C abgekühlt. Bei Zugabe von NaNO₂ (2,18 mmol, 150 mg) über eine Zeitspanne von 15 Minuten löst sich das Hydrochlorid auf, nach zehnminütigem Rühren bei -10°C wird NH₄BF₄ (2,18 mmol, 229 mg) zugegeben. Nach spätestens 15 Minuten fällt ein Feststoff aus, der abgesaugt und mit kaltem Ethanol (5 ml) und MTBE (50 ml) gewaschen wird. 3-Bromo-4-hydroxyphenyldiazoniumtetrafluoroborat wird als farbloser Feststoff mit einer Ausbeute von 88% (1,92 mmol, 550 mg) erhalten.
¹H NMR (300 MHz, DMSO) δ = 8,85 (d, J = 2,6, 1H, 2-H), 8,42 (dd, J = 2,6, 9,3, 1H, 6-H), 7,34 (d, J = 9,3, 1H, 5-H). ¹³C NMR (75 MHz, DMSO) δ = 168,4 (C-4), 136,9 (Ar), 134,6 (Ar), 118,9 (Ar), 112,3 (C-3).
IR: 3145 (m), 2235 (s, N₂), 1552 (s), 1424 (s), 1101 (s).
MS (EI) m/z = 63 (100%), 142 (30%), 172 (M⁺, 44%).

### Beispiel 4 (Heckreaktionen mit elektronenreichen Aryldiazoniumsalzen)

Es wurden Heckreaktionen der in der nachfolgenden Tabelle 1 dargestellten Aryldiazoniumsalze a-c mit Methylacrylat systematisch untersucht.

Vorangestellt werden soll die konkrete Versuchsvorschrift, mit der die Versuche durchgeführt wurden, deren Ergebnisse in der nachfolgenden Tabelle 1 angegeben sind:

Zu einer Lösung aus Methylacrylat (1,0 mmol, 86 mg, 0,09 ml), NaOAc (1,5 mmol, 123 mg) und Katalysator (2,5 mol-%, 3 mg) in absolutem Lösungsmittel (5 ml) wird das entsprechende Diazoniumsalz (0,5 mmol) gegeben und 12 Std. lang bei Raumtemperatur gerührt. Die Reaktionslösung wurde am Rotationsverdampfer eingeengt, Methyl-tert-butylether (MTBE) (10 ml) aufgenommen und mit 1 N HCI (10 ml) gewaschen. Die wässrige Phase wurde mit MTBE (30 ml) extrahiert. Die vereinigten organischen Phasen wurden über MgSO₄ getrocknet. Das Lösungsmittel wurde entfernt und das Rohprodukt chromatographisch gereinigt.

Bei den beschriebenen Versuchen wurden zwei Präkatalysatoren (Palladiumacetat und der Pd₂(dba)₃·CHC₃-Komplex) sowie zwei verschiedene Lösungsmittel (Methanol als polarprotisches und Acetonitril als polar-aprotisches Lösungsmittel) getestet. Zudem wurde die Reaktion jeweils in Gegenwart bzw. in Abwesenheit einer Base durchgeführt.

**Tabelle 1**

| Präkatalysator | Pd(OAc)₂ | | | | Pd₂(dba)₃CHCl₃ | | | |
|---|---|---|---|---|---|---|---|---|
| Lösungsmittel | MeOH | | CH₃CN | | MeOH | | CH₃CN | |
| Base | NaOAc | - | NaOAc | - | NaOAc | - | NaOAc | - |
| 1a (R=Bn) | 32 | 52 | 15 | 7 | 22 | 45 | 45 | 22 |
| 1b (R=Me) | 72 | 83 | 52 | 10 | 71 | 94 | 73 | 15 |
| 1c (R=H) | 99 | 95 | 99 | 40 | 99 | 95 | 99 | 21 |

Anmerkung: Die Zahlenwerte in den Spalten 2 ff. betreffen die jeweils erzielten Ausbeuten.

Aus der vorstehenden Tabelle lassen sich folgende Folgerungen ziehen: Die für das Benzyloxy substituierte Derivat 1a erzielten Ausbeuten sind bestenfalls mittelmäßig. In Methanol werden unter standardisierten Bedingungen in der Regel bessere Ausbeuten erzielt als in Acetonitril. Die Base hat einen erheblichen Einfluß, der allerdings je nach verwendetem Lösungsmittel entgegengesetzt ausfällt: in Methanol gelingen die Reaktionen besser, wenn keine Base zugegen ist, in Acetontril werden erheblich bessere Ausbeuten bei Anwesenheit von Basen erzielt. Es überrascht, dass in allen Versuchsreihen die besten Ergebnisse für das ungeschützte Diaryldiazoniumsalz 1c erzielt werden. Hierbei werden, unabhängig von Katalysator und Base, in Methanol quantitative Ausbeuten erhalten. In Acetontril werden quantitative Ausbeuten nur dann erhalten, wenn die Base zugegen ist.

### Beispiel 5

Wie in Abbildung 1 und Tabelle 2 zu sehen ist, wurden die drei Diazoniumsalze 1a bis c unter standardisierten Bedingungen mit drei verschiedenen Styrolen 4a bis 4c umgesetzt.

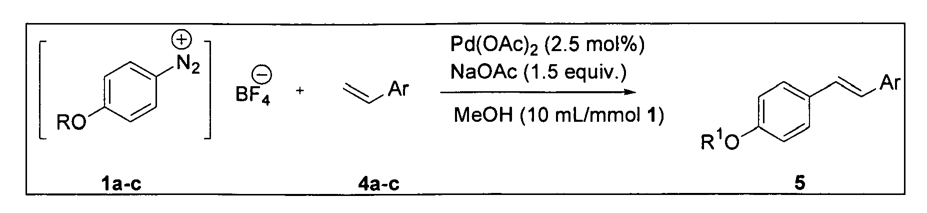

Die entsprechenden Versuche werden gemäß der folgenden Vorschrift durchgeführt: Zu einer Lösung des Styrols **4** (0,5 mmol), NaOAc (1,5 mmol, 123 mg) und Pd(OAc)₂ (2,5 mol-%, **3** mg) in absolutem Methanol (5 ml) wird das entsprechende Diazoniumsalz **1** (1,0 mmol) gegeben und 12 Std. lang bei Raumtemperatur gerührt. Die Reaktionslösung wird mit Kieselgel versetzt und am Rotationsverdampfer eingeengt. Das Rohprodukt wird chromatographisch gereinigt.

Die Ergebnisse der Versuche sind in der nachfolgenden Tabelle 2 dargestellt.

**Tabelle 2**

| | | | |
|---|---|---|---|
| Styrol→ | 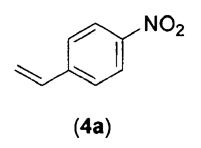 | 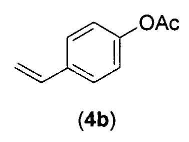 | 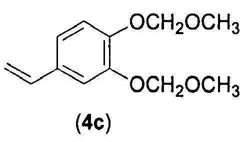 |
| Aryldiazoniumsalz↓ | | | |
| **1a** (R = Bn) | **5aa** (20) | **5ab** (18) | -- |
| **1b** (R = Me) | **5ba** (79) | **5bb** (38) | **5bc** (80) |
| **1c** (R = H) | **5ca** (98) | **5cb** (95) | **5cc** (76) |

nmerkung: Die Ausbeuten in Prozent sind in Klammern angegeben.

Es zeigt sich, dass das Aryldiazoniumsalz 1a nur schlechte Ausbeuten liefert, während mit dem ungeschützten Aryldiazoniumsalz durchweg hervorragende Ausbeuten erzielt werden können.

### Beispiel 6

Es wurde die Heckkupplung von Allylalkohol (6) mit den Diazoniumtetrafluoroboraten 1b und 1c untersucht, die in der nachfolgenden Abbildung 2 dargestellt ist.

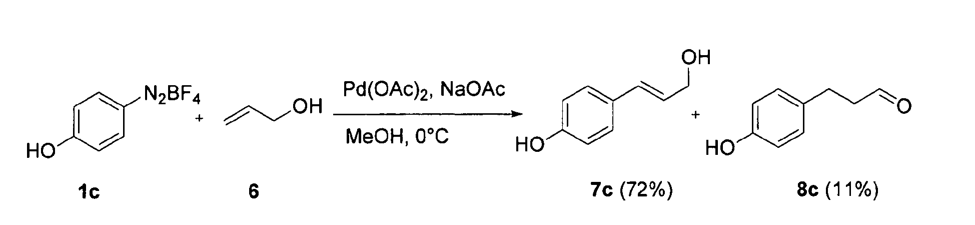

Die zugrundezulegende Reaktion wird im einzelnen wie folgt durchgeführt: Zu einer Lösung aus Allylalkohol (2,4 mmol, 0,2 ml), NaOAc (3,6 mmol, 195 mg) und Pd(OAc)₂ (5 mol-%, 14 mg) in absolutem Methanol (5 ml) wird das entsprechende Diazoniumsalz (1,2 mmol) gegeben und 6 Std. lang bei 0°C gerührt. Die Reaktionslösung wird am Rotationsverdampfer eingeengt, in MTBE (10 ml) aufgenommen und mit 1 N HCl (10 ml) gewaschen. Die wässrige Phase wird mit MTBE (30 ml) extrahiert. Die vereinigten organischen Phasen werden über MgSO₄ getrocknet. Das Lösungsmittel wird entfernt und das Rohprodukt chromatographisch (Laufmittel: cHex:MTBE 1:1) gereinigt.

Der p-Cumarylalkohol 7c wurde in 72%iger Ausbeute erhalten. Als Nebenprodukt entstand in 11%iger Ausbeute der aus einer isomerisierenden Heckkupplung resultierende Aldehyd 8c.

Bei der Durchführung der obigen Versuche hat sich noch eine interessante Erkenntnis ergeben, die sich aus den nachfolgenden Darstellungen ergibt:

Durch den Wechsel des Lösungsmittels zu Acetonitril und den Zusatz eines starken Donors (Chlorid, in Form von Tetrabutylammoniumchlorid) kann die Selektivität der Reaktion umgekehrt werden. Unter diesen Bedingungen wird in 69%iger Ausbeute nur 8c erhalten. Die Erhöhung der Isomerisierungsaktivität durch Zusatz von Halogeniden bei Heckkupplungen wurde bereits früher beschrieben, jedoch nicht für Diazoniumsalze als Kupplungspartner ("Tetrahedron Lett." 1989, 30, 2603-2606). Bei Verwendung des Methoxyderivats 1b wird in Methanol der zu 7c analoge Alkohol 7b erhalten. In Acetonitril in Gegenwart von Tetrabutylammoniumchlorid kann hingegen bei Verwendung von 1b kein Produkt isoliert werden, so dass auch in diesem Beispiel von einer insgesamt höheren Aktivität des ungeschützten para-Hydroxyphenyldiazoniumsalzes 1c ausgegangen werden kann.

### Beispiel 7

Eine Suzuki-Kupplung des Phenyltrifluoroborats 1c wurde gemäß Abbildung 3 mit Kalium Phenyltrifluoroborat in Methanol unter Verwendung von Pd(OAc)₂ als Katalysator zu (1,1'-Biphenyl)-4-ol umgesetzt. Der Alkohol wurde in 51 % Ausbeute erhalten.

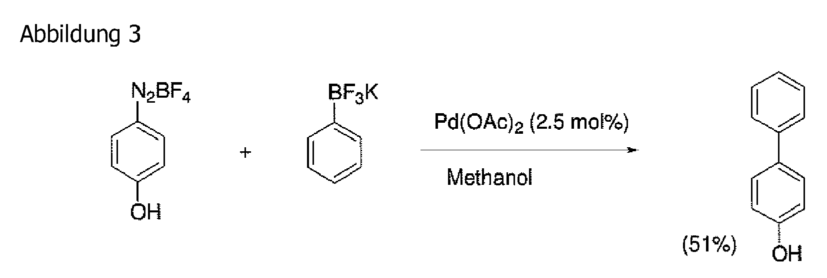

Bei der Reaktion tritt im Gegensatz zu dem in "Tetrahedron Lett.", 1979, 657-660 geschilderten Verfahren kein Regioselektivitätsproblem auf. Ebenso wird nur eine kata-lytische Menge Metallsalz verwendet und die Ausbeute ist wesentlich höher.

## Patentansprüche

1. Phenolisches Diazoniumsalz der allgemeinen Formel (I), 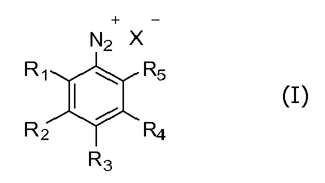 **dadurch gekennzeichnet, dass** mindestens einer von R¹, R², R³, R⁴ oder R⁵ eine Hydroxygruppe ist und die anderen Substituenten, unabhängig voneinander, Wasserstoff, Halogen, eine Alkyl-, Alkenyl-, Aryl-, Alkoxy-, Aryloxy-, Nitro-, Cyano-, Hydroxy-, Acetyl- und/oder Diazogruppen darstellen, und X BF₄, Cl, F, SO₃CH₃, CO₂CH₃, PF₆, ClO₂CH₃ oder ClO₄ darstellen.

2. Phenolisches Diazoniumsalz gemäß Anspruch 1, **dadurch gekennzeichnet, dass** die Alkylgruppe eine geradkettige oder verzweigte (C₁-C₆)-Alkylgruppe, eine (C₃-C₇)-Cycloalkylgruppe, die Alkenylgruppe eine geradkettige oder verzweigte (C₁-C₆)-Alkenylgruppe, die Alkoxygruppe eine geradkettige oder verzweigte (C₁-C₆)-Alkyloxygruppe, die Arylgruppe eine Benzyl-, 4-Methoxybenzyl-, 4-Nitrobenzyl-, Phenyl- oder 4-Methoxyphenylgruppe darstellt und Halogen Fluor, Chlor oder Brom darstellen.

3. Phenolisches Diazoniumsalz gemäß Anspruch 1 oder 2, **dadurch gekennzeichnet, dass** R³ eine Hydroxygruppe ist.

4. Phenolisches Diazoniumsalz gemäß Anspruch 3, **dadurch gekennzeichnet, dass** R¹, R², R⁴ und R⁵ jeweils Wasserstoffe sind.

5. Phenolisches Diazoniumsalz gemäß einem der Ansprüche 1 bis 3, **dadurch gekennzeichnet, dass** es mindestens einen weiteren Sustituenten enthält, der kein Wasserstoff ist.

6. Phenolisches Diazoniumsalz gemäß einem der Ansprüche 1 bis 5, **dadurch gekennzeichnet, dass** X Tetrafluoroborat ist.

7. Verfahren zur Herstellung eines phenolischen Diazoniumsalzes der im Anspruch 1 dargestellten allgemeinen Formel (I), **dadurch gekennzeichnet, dass** als Ausgangsverbindung ein benzolisches Amid, das bis auf die Diazofunktion die gleichen Substituenten R¹, R², R³, R⁴ oder R⁵ aufweist, eingesetzt wird und dieses zunächst hydrolytisch gespalten und das so erhaltene Amin mit einem anorganischen Nitritsalz diazotiert und gegebenenfalls isoliert wird.

8. Verfahren gemäß Anspruch 7, **dadurch gekennzeichnet, dass** das diazotierte Zwischenprodukt durch Zugabe eines Komplexanionsalzes mit einem Anion in Form von BF₄⁻, PF₆ und/oder ClO4⁻ in das Diazoniumsalz überführt wird.

9. Verfahren gemäß Anspruch 7 oder 8, **dadurch gekennzeichnet, dass** die hydrolytische Spaltung mit einer Mineralsäure oder Fluoroborsäure durchgeführt wird.

10. Verfahren gemäß einem der Ansprüche 7 bis 9, **dadurch gekennzeichnet, dass** die hydrolytische Spaltung in einem alkoholischen Lösungsmittel, das eine Mineralsäure, insbesondere Chlorwasserstoffsäure, oder Fluoroborsäure enthält, durchgeführt wird, wobei der Alkohol insbesondere ein C₁-C₄-Alkohol darstellt.

11. Verfahren gemäß Anspruch 10, **dadurch gekennzeichnet, dass** als alkoholisches Lösungsmittel Methanol, Ethanol, n-Propanol und/oder Isopropanol eingesetzt werden.

12. Verfahren gemäß einem der Ansprüche 7 bis 11, **dadurch gekennzeichnet, dass** die hydrolytische Spaltung des Amids bei einer Temperatur zwischen etwa 20 und 110°C, insbesondere zwischen etwa 50 und 80°C, durchgeführt wird.

13. Verfahren gemäß einem der Ansprüche 7 bis 12, **dadurch gekennzeichnet, dass** die Diazotierung zwischen etwa -10 und +10°C, insbesondere zwischen etwa -5 und +5°C durchgeführt wird.

14. Verfahren gemäß einem der Ansprüche 7 bis 13, **dadurch gekennzeichnet, dass** die hydrolytische Spaltung und die Diazotierung in der gleichen Reaktionsmischung ohne zwischengeschaltete Isolierung des Amins durchgeführt werden.

15. Verwendung eines phenolischen Diazoniumsalzes gemäß einem der Ansprüche 1 bis 6 als Substrat in einer Kreuzkupplungsreaktion.

16. Verwendung gemäß Anspruch 15, **dadurch gekennzeichnet, dass** die Kreuzkupplungsreaktion eine Heck-, Suzuki-, Stille-, Negishi-, Hiyama- oder Kumada-Kupplung ist.

17. Verwendung nach Anspruch 15 oder 16, **dadurch gekennzeichnet, dass** die Kreuzkupplungsreaktion nach einem Verfahren durchgeführt wird, umfassend
(a) das Mischen eines in den Ansprüchen 1 bis 6 definierten phenolischen Diazoniumsalzes mit einem Kupplungspartner in Anwesenheit eines Katalysators, wobei der Kupplungspartner eine Verbindung der allgemeinen Formel (II) 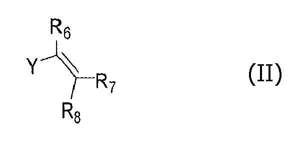 darstellt, R⁶, R⁷ und R⁸, die gleich oder verschieden sind, Wasserstoff, Carboxyalkylreste, Carboxyarylreste, Alkylreste, Arylreste, Alkoxyreste, Aryloxyreste, wobei die Reste jeweils Si, N, S, O, und oder Halogen-Atome enthalten können, darstellen oder R⁶ und R⁷ mit der Doppelbindung einen aromatischen Ring bilden, der mit R⁸ und ein bis vier weiteren Substituenten, unabhängig voneinander, ausgewählt aus der Gruppe bestehend aus einer geradkettigen oder verzweigten (C₁-C₆)-Alkylgruppe, einer (C₃-C₇)-Cycloalkylgruppe, einer geradkettigen oder verzweigten (C₁-C₆)-Alkenylgruppe, einer geradkettigen oder verzweigten (C₁-C₆)-Alkoxygruppe, Halogen, der Hydroxygruppe, einer Amino-, Di(C₁-C₆)-alkylamino-, Nitro-, Acetyl-, Cyan-, Benzyl-, 4-Methoxybenzyl-, 4-Nitrobenzyl-, Phenyl- und 4-Methoxyphenylgruppe, versehen sein kann und Y = H, -B(OR)₂, -SnR₃, -ZnR, -SiR₃ oder Mg (Halogen) ist,
(b) Umsetzen des phenolischen Diazoniumsalzes mit dem Kupplungspartner und der Bildung des kreuzgekuppelten Produkts.

18. Verwendung gemäß Anspruch 17, **dadurch gekennzeichnet, dass** als Katalysator ein Übergangsmetall-Katalysator, insbesondere ein Palladium-Katalysator eingesetzt wird.

19. Verwendung gemäß Anspruch 18, **dadurch gekennzeichnet, dass** als Katalysator Pd(OAc)₂ oder Pd₂(dba)₃CHCl₃ eingesetzt wird.

20. Verwendung gemäß einem der Ansprüche 17 bis 19, **dadurch gekennzeichnet, dass** vor der Durchführung des Schritts (b) dem Reaktionsgemisch eine Base, insbesondere eine sich in dem Reaktionsgemisch gut lösende Base zur Abpufferung der im Schritt (b) entstehenden Säure zugegeben wird.

21. Verwendung gemäß einem der Ansprüche 17 bis 20, **dadurch gekennzeichnet, dass** die Umsetzung in einem Lösungsmittel durchgeführt wird, das auf Methanol, Ethanol, Acetonitril und/oder Wasser beruht.

22. Verfahren gemäß einem der Ansprüche 17 bis 21, **dadurch gekennzeichnet, dass** die Umsetzung bei einer Temperatur von etwa -10 bis 60°C, insbesondere von etwa 20 bis 30°C durchgeführt wird.
